# EUROPEAN PATENT SPECIFICATION

(11) **EP 1 016 657 B1**
(45) Date of publication and mention of the grant of the patent: **30.01.2002**
(21) Application number: 99203800.0
(22) Date of filing: 15.11.1999
(51) Int. Cl.: C07C 409/26, C07C 407/00

(54) **Method for producing peracetic acid**
Verfahren zur Herstellung von Peressigsäure
Procédé pour la préparation de l'acide peracétique

(30) Priority: 15.12.1998 EP 98850194
(43) Date of publication of application: 05.07.2000
(73) Proprietor: AKZO NOBEL N.V., 6800 SB Arnhem (NL); Eka Chemicals AB, 445 80 Bohus (SE)
(72) Inventor: Persson, Hakan, 442 91 Romelunda (SE); Hallberg, Richard, 442 75 Lycke (SE); Pudas, Roland, 449 70 Nödinge (SE)
(74) Representative: Jönsson, Christer

(56) References cited:
- EP-A- 0 641 777
- EP-A- 0 789 016
- FR-A- 1 432 773
- CHEMICAL ABSTRACTS, vol. 116, no. 14, 6 April 1992 (1992-04-06) Columbus, Ohio, US; abstract no. 131613, BATOG A E ET AL: "Method for preparing aqueous peracetic acid" XP002099898 & SU 1 668 358 A (USSR)

## Description

The present invention relates to a method for producing peracetic acid by reacting hydrogen peroxide and acetic acid in an aqueous reaction medium, from which peracetic acid is continuously distilled off.

Peracetic acid in aqueous solution can be used as disinfectant or for bleaching various materials and is especially suited for environment-friendly bleaching of cellulose pulp.

Peracetic acid can be produced by reacting hydrogen peroxide and acetic acid in an aqueous reaction medium and distilling off peracetic acid, which is described in US 3264346. This process has later been considerably improved and made suitable for commercial use by the invention described in EP 789016. However, there is still need for further improvements to increase the production capacity and/or the purity of the product. Further, it is difficult to control the process and maintain optimal reaction conditions.

The main impurity in the peracetic acid product is acetic acid. By maintaining a comparatively low concentration of acetic acid in the reaction medium it has been found possible to minimise this impurity, without adversely affecting the production rate. Low concentration of acetic acid also enables higher production capacity since the distillation can be performed with lower reflux ratio. In any event, it is desirable to avoid large variations in the concentration of acetic acid in the reaction medium.

Peracetic acid decomposes easily to acetic acid and oxygen, and sometimes this occurs in the distillation column resulting in increasing acetic acid concentration in the reaction medium. Therefore, it is extremely difficult to maintain steady state conditions in the reaction medium.

It has now surprisingly been found possible to control the concentration of acetic acid by maintaining the temperature substantially constant and regulating the feed of acetic acid based on the temperature.

Thus, the invention concerns a method for producing peracetic acid by continuously feeding hydrogen peroxide and acetic acid to an aqueous reaction medium in a reactor, reacting said hydrogen peroxide and acetic acid in the reaction medium to yield peracetic acid and continuously distilling off said peracetic acid. The feed of acetic acid is regulated based on a set value for the temperature of the reaction medium. Suitably, the concentration of acetic acid in the reaction medium is maintained from about 0.5 to about 8 wt%, preferably from about 2 to about 4 wt%, while the concentration of hydrogen peroxide suitably is maintained from about 10 to about 35 wt%, preferably from about 20 to about 30 wt%. Preferably, the molar ratio of hydrogen peroxide to acetic acid in the reaction medium is from about 5:1 to about 35:1, most preferably from 8:1 to about 15:1. The suitable set value for the temperature normally corresponds to the bubble point and depends on the exact composition of the reaction medium as well as the pressure. Suitably, a set value from about 40 to about 70°C, preferably from about 50 to about 60°C is used.

Normally the reaction medium also contains peracetic acid, preferably in an amount from about 0.5 to about 8 wt%, most preferably from about 2 to about 4 wt%. In order to increase the production rate the concentration thereof is preferably maintained below the equilibrium concentration.

The distillation is suitably operated to obtain as a product an aqueous solution containing peracetic acid and less than about 10 wt%, preferably less than 5 wt%, most preferably less than about 2 wt% acetic acid. Further, the product is preferably substantially free from hydrogen peroxide and mineral acid. This can normally be achieved if the distillation is operated with a suitable reflux ratio, defined as reflux mass flow to product mass flow, from about 0.3:1 to about 2:1, preferably from about 0.3:1 to about 1.5:1.

Suitably, thermal energy is supplied to the reaction medium in an amount exceeding about 0.2 kW/kg reaction medium, preferably in an amount from about 0.5 to about 5 kW/kg, most preferably from about 0.8 to about 5 kW/kg, particularly most preferably from about 1 to about 3 kW/kg.

Suitably, the reaction medium also contains an acid catalyst, preferably one or more mineral acids in an amount from about 10 to about 25 wt%, most preferably from about 15 to about 20 wt%. Examples of usable mineral acids are sulphuric acid and phosphoric acid. In order to reduce the decomposition of peracetic acid and/or hydrogen peroxide, the reaction medium suitably also contains one or more stabilisers such as phosphonic acids or salts thereof and/or dipicolinic acid or derivatives thereof. Usable phosphonic acids include e.g. 1-hydroxyethylidene-1,1-diphosphonic acid, 1-aminoethane-1,1-diphosphonic acid, aminotri-(methylene phosphonic acid), ethylenediamine-tetra(methylene phosphonic acid), hexamethylenediamine-tetra(methylene phosphonic acid), diethylenetriamine-penta(methylene phosphonic acid), diethylenetriamine-hexa(methylene phosphonic acid), dimethylamino methanediphosphonic acid, aminoacetic acid-N,N-dimethylene phosphonic acid, 3-aminopropane-1-hydroxy-1,1-diphosphonic acid, 2-phosphonobutane-1,2,4-tricarboxylic acid, phosphonosuccinic acid, 1-phosphono--1-methylsuccinic acid and 1-amino-phenylmethane diphosphonic acid.

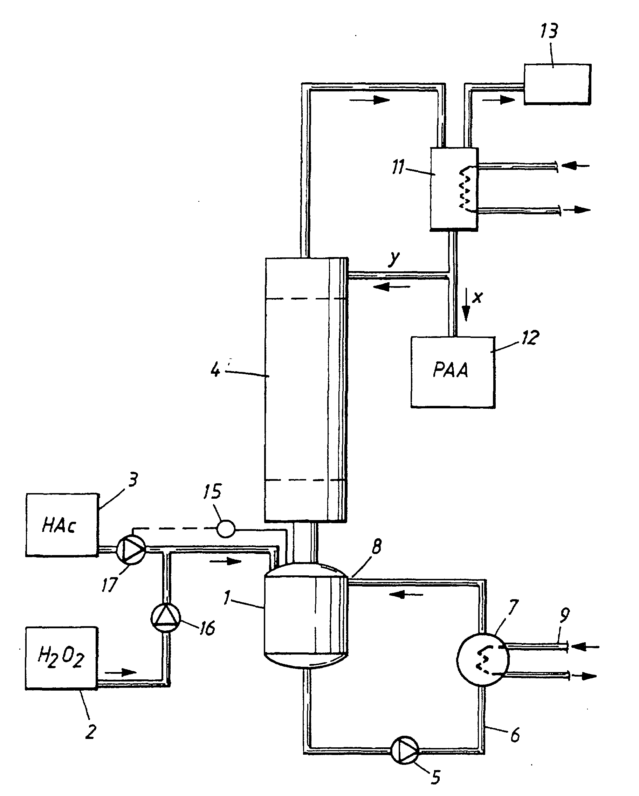

The invention will now be described in more detail with reference to the accompanying Figure, which schematically illustrates a process for producing peracetic acid. However, the invention is not restricted to the embodiment shown, but only by the scope of the claims.

A reactor 1 connected to a distillation column 4 holds a reaction medium made up of an aqueous solution containing hydrogen peroxide (H₂O₂), acetic acid (HAc), peracetic acid (PAA), a mineral acid such as sulphuric acid, and a stabiliser such as a phosphonic acid. The reactor 1 is continuously supplied with aqueous solutions of hydrogen peroxide from a tank 2 and acetic acid from a tank 3 via feed pumps 16, 17. The temperature of the reaction medium in the reactor 1 is measured with means therefore 15 and the feed pump 17 for acetic acid is regulated based on said temperature. The feed rate of hydrogen peroxide is preferably regulated based on the volume of the reaction medium in the reactor 1 but may also be maintained substantially constant. Depending on the concentrations of the other feed streams it is also possible to supply fresh water to the reactor (not shown). A subatmospheric pressure and a temperature sufficient for peracetic acid and water to be withdrawn through the distillation column 4 prevail in the reactor 1, which normally implies that the reaction medium is boiling. Preferably the pressure in the reactor 1 is maintained substantially constant and is suitably within the range from about 40 to about 250 mbar, preferably from about 60 to about 150 mbar.

The temperature in the reactor 1 is maintained at the bubble point, which depends on the pressure and the exact composition of the reaction medium. In order to optimise the production of high quality peracetic acid, it is preferred to keep both the pressure and the composition substantially constant, although it is difficult to avoid that peracetic acid may decompose to acetic acid in the distillation column 4 or in the reactor 1. However, this causes a decrease in the bubble point and thereby also the temperature of the reaction medium that is measured with the means15 therefore. A signal is then sent to the feed pump 17 to decrease the supply of acetic acid so acetic acid in the reaction medium is brought down to the desired level again, which is indicated by the temperature. The transformation of temperature to acetic acid feed rate can be accomplished with any kind of commercially available control system. It has surprisingly been found that this procedure works out well in spite of having such a complex reaction medium containing more than two components.

Sufficient thermal energy is supplied to the reactor 1 by circulating the reaction medium with the aid of a pump 5 in a loop 6 through a heat exchanger 7, where it is heated by means of a heat source 9 such as hot water, before being recycled to the reactor 1 through an inlet 8. Suitably, the static height of the liquid and thereby the pressure is sufficiently high so no boiling takes place in the loop 6 until heated reaction medium has entered the reactor 1 through the inlet 8, in spite of the temperature increase which suitably is less than about 30°C, preferably less than about 20°C, most preferably less than about 15°C. The temperature of the reaction medium immediately after heating is preferably from about 5 to about 15°C higher than in the reactor 1, most preferably from about 8 to about 12°C higher than in the reactor 1. Preferably, the temperature of the heated reaction medium in the circulation loop 6 does not exceed about 80°C, most preferred not about 70°C. Preferably, the reaction medium in the reactor 1 is circulated through the loop 6 from about 30 to about 200 times per hour, most preferably from about 70 to about 150 times per hour through the circulation loop 6 and the heat exchanger 7. This makes it possible to supply great amounts of thermal energy to the reactor 1 without unacceptable decomposition of peracetic acid or hydrogen peroxide.

In the distillation column 4, the peracetic acid is separated from the other components in the reaction medium, and an aqueous solution of from about 20 to about 60 wt%, preferably from about 30 to about 50 wt% peracetic acid suitably containing less than about 10 wt%, preferably less than about 5 wt%, most preferably less than about 2 wt% of acetic acid and most preferably being substantially free from hydrogen peroxide and mineral acid, is caused to condense in a condenser 11. Part of the condensate x is conducted to a product tank 12 while the remainder y is recycled to the distillation column 4 to form a reflux flow. In order to obtain a sufficiently pure product it is normally suitable to operate with a reflux ratio y/x based on mass flow from about 0.3:1 to about 2:1, preferably from about 0.3:1 to about 1.5:1. A vacuum source 13 is connected to the condenser such that a suitable pressure prevails in the column 4 and the reactor 1. To the top of the distillation column 4 a stabiliser can be supplied, for instance one of the above-mentioned phosphonic acids or a salt thereof, wherein part of the stabiliser normally accompanies the product to the tank 12 while the remainder is conveyed downwards with the reflux flow and distributed in the column 4 and the reactor 1.

The process equipment, such as reactor, distillation column, piping, heat exchangers, tanks, etc., are suitably made of a corrosion-proof material such as aluminium or stainless steel, for instance SS 2343, 2353, 2304, 2305, 254 SMO or Hasteloy™. Other usable materials are tantalum, glass or vitreous enamel, impregnated graphite, fluoroplastics, such as PTFE, PVDF, PFA or FEP, fluoroplastic-coated materials, polyethylene, polypropylene, siliceous carbide or ceramic materials.

## Claims

1. A method for producing peracetic acid by continuously feeding hydrogen peroxide and acetic acid to an aqueous reaction medium in a reactor, reacting said hydrogen peroxide and acetic acid in the reaction medium to yield peracetic acid and continuously distilling off said peracetic acid, **characterised in that** the feed of acetic acid is regulated based on a set value for the temperature of the reaction medium.

2. A method as claimed in claim 1, **characterised in that** the concentration of acetic acid in the reaction medium is maintained from 0.5 to 8 wt%.

3. A method as claimed in any one of the claims 1-2, **characterised in that** the set value for the temperature of the reaction medium is from 40 to 70°C.

4. A method as claimed in any one of the claims 1-3, **characterised in that** the concentration of hydrogen peroxide in the reaction medium is maintained from 10 to 35 wt%.

5. A method as claimed in any one of the claims 1-4, **characterised in that** the molar ratio of hydrogen peroxide to acetic acid in the reaction medium is maintained from 5:1 to 35:1.

6. A method as claimed in any one of the claims 1-5, **characterised in that** the reaction medium contains hydrogen peroxide, acetic acid, peracetic acid and a mineral acid.

7. A method as claimed in claim 6, **characterised in that** the mineral acid is sulfuric acid.

8. A method as claimed in any one of the claims 1-7, **characterised in that** the pressure in the reactor is maintained from 40 to 250 mbar.

9. A method as claimed in any one of the claims 1-8, **characterised in that** the distillation is operated with a reflux ratio, defined as reflux mass flow to product mass flow, from 0.3:1 to 1.5:1.

10. A method as claimed in any one of the claims 1-9, **characterised in that** the distillation is operated to obtain as a product an aqueous solution containing peracetic acid and less than 5 wt% of acetic acid.

## Patentansprüche

1. Verfahren zur Herstellung von Peressigsäure durch kontinuierliche Einspeisung von Wasserstoffperoxid und Essigsäure in ein wässriges Reaktionsmedium in einem Reaktor, wobei das Wasserstoffperoxid und die Essigsäure im Reaktionsmedium unter Bildung von Peressigsäure umgesetzt werden und die Peressigsäure kontinuierlich abdestilliert wird, **dadurch gekennzeichnet, dass** die Einspeisung der Essigsäure basierend auf einem festgelegten Wert für die Temperatur des Reaktionsmediums geregelt wird.

2. Verfahren nach Anspruch 1, **dadurch gekennzeichnet, dass** die Konzentration der Essigsäure im Reaktionsmedium bei 0,5 bis 8 Gewichts-% gehalten wird.

3. Verfahren nach einem der Ansprüche 1 bis 2, **dadurch gekennzeichnet, dass** der festgelegte Wert für die Temperatur des Reaktionsmediums 40 bis 70 °C beträgt.

4. Verfahren nach einem der Ansprüche 1 bis 3, **dadurch gekennzeichnet, dass** die Konzentration des Wasserstoffperoxids im Reaktionsmedium bei 10 bis 35 Gewichts-% gehalten wird.

5. Verfahren nach einem der Ansprüche 1 bis 4, **dadurch gekennzeichnet, dass** das Molverhältnis von Wasserstoffperoxid zu Essigsäure im Reaktionsmedium bei 5:1 bis 35:1 gehalten wird.

6. Verfahren nach einem der Ansprüche 1 bis 5, **dadurch gekennzeichnet, dass** das Reaktionsmedium Wasserstoffperoxid, Essigsäure, Peressigsäure und eine Mineralsäure enthält.

7. Verfahren nach Anspruch 6, **dadurch gekennzeichnet, dass** die Mineralsäure Schwefelsäure ist.

8. Verfahren nach einem der Ansprüche 1 bis 7, **dadurch gekennzeichnet, dass** der Druck im Reaktor bei 40 bis 250 mbar gehalten wird.

9. Verfahren nach einem der Ansprüche 1 bis 8, **dadurch gekennzeichnet, dass** die Destillation mit einem Rückflussverhältnis, definiert als Rückfluss-Massenstrom zu Produkt-Massenstrom, von 0,3:1 bis 1,5:1 ausgeführt wird.

10. Verfahren nach einem der Ansprüche 1 bis 9, **dadurch gekennzeichnet, dass** die Destillation so ausgeführt wird, dass als Produkt eine wässrige Lösung erhalten wird, die Peressigsäure und weniger als 5 Gewichts-% Essigsäure enthält.

## Revendications

1. Procédé de production d'acide peracétique, dans lequel on introduit de manière continue du peroxyde d'hydrogène et de l'acide acétique dans un milieu de réaction aqueux, contenu dans un réacteur, on fait réagir ledit peroxyde d'hydrogène et ledit acide acétique dans le milieu de réaction pour former de l'acide peracétique et on sépare de manière continue par distillation ledit acide peracétique, procédé **caractérisé en ce que** l'on règle la quantité introduite d'acide acétique en se basant sur une valeur fixée pour la température du milieu de réaction.

2. Procédé selon la revendication 1, **caractérisé en ce que** la concentration de l'acide acétique dans le milieu de réaction est maintenue à une valeur comprise dans l'intervalle allant de 0,5 à 8 % en poids.

3. Procédé selon la revendication 1 ou 2, **caractérisé en ce que** la valeur fixée pour la température du milieu de réaction est comprise dans l'intervalle allant de 40 à 70 °C.

4. Procédé selon l'une quelconque des revendications 1 à 3, **caractérisé en ce que** l'on maintient la concentration du peroxyde d'hydrogène dans le milieu de réaction à une valeur comprise dans l'intervalle allant de 10 à 35 % en poids.

5. Procédé selon l'une quelconque des revendications 1 à 4, **caractérisé en ce que** l'on maintient le rapport molaire du peroxyde d'hydrogène à l'acide acétique dans le milieu de réaction à une valeur comprise dans l'intervalle allant de 5:1 à 35:1.

6. Procédé selon l'une quelconque des revendications 1 à 5, **caractérisé en ce que** le milieu de réaction contient du peroxyde d'hydrogène, de l'acide acétique, de l'acide peracétique et un acide minéral.

7. Procédé selon la revendication 6, **caractérisé en ce que** l'acide minéral est l'acide sulfurique.

8. Procédé selon l'une quelconque des revendications 1 à 7, **caractérisé en ce que** la pression dans le réacteur est maintenue à une valeur allant de 40 à 250 mbar.

9. Procédé selon l'une quelconque des revendications 1 à 8, **caractérisé en ce que** l'on réalise la distillation à un taux de reflux de 0,3:1 à 1,5:1, le taux de reflux étant défini comme étant le rapport du courant de reflux en masse au courant de produit en masse.

10. Procédé selon l'une quelconque des revendications 1 à 9, **caractérisé en ce que** l'on réalise la distillation de façon à obtenir comme produit une solution aqueuse contenant de l'acide peracétique et moins de 5 % en poids d'acide acétique.
